# EUROPÄISCHE PATENTSCHRIFT

(11) **EP 0 594 946 B1**
(45) Veröffentlichungstag und Bekanntmachung des Hinweises auf die Patenterteilung: **11.09.1996**
(21) Anmeldenummer: 93110327.9
(22) Anmeldetag: 29.06.1993
(51) Int. Cl.: A61B 17/28

(54) **Greif- und/oder Schneidinstrument für endoskopische Zwecke**
Gripping and/or cutting device for endoscopic surgery
Instrument endoscopique de coupe et/ou pince endoscopique

(30) Priorität: 22.07.1992 DE 4224226; 22.07.1992 DE 4235023
(43) Veröffentlichungstag der Anmeldung: 04.05.1994
(73) Patentinhaber: FRIATEC AG, D-68229 Mannheim (DE)
(72) Erfinder: Weber, Robert, Chino Hills, California 91709 (US); Schaber, Georg, Venice, California 90291 (US); Frings, Lars, D-5630 Remscheid (DE)
(74) Vertreter: Goddar, Heinz J., Dr.

(56) Entgegenhaltungen:
- EP-A- 0 119 405
- EP-A- 0 313 820
- EP-A- 0 484 671
- US-A- 4 369 788
- US-A- 4 813 407

## Beschreibung

Die Erfindung betrifft ein Greif- und/oder Schneidinstrument für endoskopische Zwecke, mit den Merkmalen des Oberbegriffs von Patentanspruch 1.

Endoskopische Instrumente, die die operative Behandlung von Gewebe- oder Gefäßverletzungen vereinfachen sollen, sind in vielfältigen Varianten, insbesondere in ihrer Ausgestaltung als Greif- bzw. Schneidinstrument bekannt. So Zeigt das US-Geschmacksmuster 274,096 ein arthroskopisches Schneidinstrument, dessen Greifarme mit Hilfe eines über die Schenkel betätigbaren Mechanismus geöffnet und geschlossen werden können.

Ein weiteres Instrument ist aus der US-A 4,890,615 bekannt. Mit dem dort beschriebenen Instrument sollen insbesondere Nähte innerhalb des menschlichen Körpers gebildet werden. Dazu wird das Gewebe zwischen relativ zueinander bewegbaren Backen eingeklemmt, von denen eine als Hohlnadel ausgebildet ist, durch welche der Nähfaden gezogen wird. Die Backen lassen sich wiederum durch einen über die Schenkel betätigbaren Mechanismus öffnen und schließen, wobei eine Zugkraft auf die Übertragungsstange aufgebracht wird, wenn die Backen geschlossen werden sollen. Der an dem Auge eines der Schenkel vorgesehene Stützansatz dient als Auflage für einen Finger und wirkt somit unterstützend für die Schließbewegung der Backen.

Ein chirugisches Instrument, bei dem die Greif- und/oder Schneideinrichtungen bei Druck auf die Schubstange schließen, ist aus der EP-A 0 119 405 bekannt. Maßnahmen, welche das präzise Führen des Instruments verbessern, sind jedoch nicht angegeben. Dasselbe gilt für das Instrument gemäß der US-A 4,369,788, die zur Bildung des Oberbegriffes von Patentanspruch 1 herangezogen wurde.

Es ist die Aufgabe der vorliegenden Erfindung, ein Instrument für endoskopische Zwecke zu schaffen, insbesondere ein Greif-und Schneidinstrument, welches präzise führbar ist und mit dem ggfls. extrem saubere Schnitte möglich sind, bei denen das umgebende Gewebe nicht verletzt wird.

Diese Aufgabe wird von einem Greif- und/oder Schneidinstrument mit den Merkmalen von Patentanspruch 1 gelöst. Vorteilhafte Ausgestaltungen sind Gegenstand der Unteransprüche.

Erfindungsgemäß sind zwischen einer Öffnungsstellung und einer Schließstellung gegeneinander bewegbare Schneiden vorgesehen, von denen eine an einem distalen Ende der Übertragungsstange so angelenkt ist, daß die Öffnungsstellung bei Aufbringen einer Zugkraft auf die Übertragungsstange erreicht wird, während die Schließstellung bei Aufbringen einer Druckkraft auf die Übertragungsstange erreicht wird, und daß der Stützansatz in Richtung auf den zweiten Schenkel gekrümmt ist. Da die Übertragungsstange auf Druck wirkt, können größere Kräfte als bisher möglich übertragen werden.

Mit einem derart wirkenden Mechanismus kann auch eine einfache Überlastsicherung vorgesehen werden, der beispielsweise aus einem Scherstift besteht, welcher bricht, wenn die aufgebrachten bzw. aufzubringenden Kräfte einen Grenzwert überschreiten. Zudem wird der Zusammenbau des erfindungsgemäßen Instrumentes erheblich vereinfacht, da es nicht erforderlich ist, eine präzise Aufhängung für ein Zugorgan vorzusehen. Durch die erfindungsgemäß vorgesehene Ausgestaltung des Stützansatzes wird erreicht, daß die Relativbewegung der Schenkel auch bei ihrer Öffenbewegung kontrollierbar ist. Bisher bekannte Ausgestaltungen von endoskopischen Instrumenten weisen einen Stützansatz auf, der die Schließbewegung der Schenkel erleichtert. Diese Bewegung kann jedoch aus ergonomischen und physiologischen Gründen wegen der für eine schließende Bewegung ausgelegte Muskulatur der Hand immer kontrollierbar durchgeführt werden, wohingegen dieses für die Öffenbewegung der Schenkel nicht ohne weiteres gilt. Nunmehr ist die Möglichkeit gegeben, daß die Muskulatur eines weiteren Fingers des Benutzers an der Öffenbewegung mitwirken kann.

Vorteilhaft ist die Übertragungsstange an einem fest mit dem entsprechenden Schenkel verbundenen, um die Achse schwenkbaren Lagerelement angelenkt, dessen Schwenkwinkel begrenzt ist. Auf diese Weise können sowohl die Öffenbewegung als auch die Schließbewegung der Schneiden in einem gewünschten Maße eingeschränkt werden.

Die Begrenzung des Schwenkwinkels kann auf einfache Weise erreicht werden, wenn das Lagerelement eine Ausnehmung aufweist, in die Anschlagstift eingreift. Die Ausnehmung kann dabei eine segmentartige Aussparung des Lagerelementes in dessen Kantenbereich sein, was fertigungstechnische Vorteile bietet, sie kann aber auch durch ein gegebenenfalls gekrümmtes Langloch im Inneren des Lagerelementes gebildet sein.

Die Handhabung des Instrumentes wird weiter vereinfacht, wenn das Lagerelement beidseitig mit jeweils einer Scheibe aus reibungsminderndem Material wie Polytetrafluoräthylen versehen ist. Damit ist eine komplette Lagerung auf beiden Seiten der Scheibe in reibungsminderndem Material gewährleistet.

Nach einer besonders vorteilhaften Ausgestaltung der Erfindung ist vorgesehen, daß wenigstens eine der Schneiden gezahnt ist. Hierdurch ergibt sich die Möglichkeit, wie weiterhin erfindungsgemäß vorgesehen, zwischen den schneidenden Zähnen eine Vielzahl von aneinandergereihten, bogenförmigen schneidenden Teilbereichen auszubilden. Zu bearbeitendes Gewebe kann jetzt zu Schnittbeginn nicht mehr aus dem Bereich zwischen den Schneiden rutschen, und das einmal gefaßte Gewebe wird zuverlässig und mit gleichmäßigem Kraftaufwand geschnitten. Der Nachteil einer einheitlichen Schneide, bei der bei Fortführen des Schnittes infolge des Nachlassens der Hebelwirkung der Schneidwiderstand immer schwerer überwunden werden kann, wird vollständig vermieden.

Da das gesamte Instrument zweckmäßigerweise gekapselt ist, sollte auch die Übertragungsstange in einem langgestreckten Rohr untergebracht sein, in welchem eine die Übertragungsstange im wesentlichen umgebende Buchse aus reibungsminderndem Material wie Polytetrafluoräthylen vorgesehen ist. Hierdurch wird die gleichmäßige Bewegung der Übertragungsstange bei ihrer Bewegung längs des Rohres gewährleistet, was wiederum die Handhabbarkeit des Instrumentes erleichtert.

Bei einer weiteren Ausführungsform der Erfindung ist eine Verriegelungseinrichtung vorgesehen, welche den ersten Schenkel und den zweiten Schenkel in einer einstellbaren relativen Winkelposition hält. Damit kann das Instrument als Faßzange verwendet werden, welche, auch abhängig von der Dicke des gegriffenen Gewebes, in der Greifposition verriegelt werden kann, so daß das einmal gegriffene Gewebe vom Instrument selbst fest gehalten wird, ohne daß der Operateur die Greifposition ständig aufrechterhalten muß.

Vorteilhaft weist die Verriegelungseinrichtung einen ersten Arm auf, dessen eines Ende an einem der Schenkel angelenkt ist und dessen anderes Ende eine Klinkenvorrichtung trägt, die mit einer zweiten, kongruent ausgebildeten Klinkenvorrichtung an dem anderen der Schenkel in Eingriff bringbar ist.

Bevorzugt ist die Klinkenvorrichtung an der Verriegelungseinrichtung eine Zahnung; auch kann die zweite Klinkenvorrichtung eine Zahnung aufweisen.

Weiter bevorzugt ist in dem die zweite Klinkenvorrichtung aufweisenden Schenkel eine Durchgangsöffnung ausgebildet, in die die zweite Klinkenvorrichtung eingelassen ist und die so dimensioniert ist, daß zumindest die Klinkenvorrichtung am Arm durch den freibleibenden Teil der Durchgangsöffnung geführt werden kann.

Vorteilhaft ist vorgesehen, daß die Verriegelungseinrichtung einen Betätigungshebel aufweist, mittels dessen der Arm um seinen Anlenkpunkt verschwenkbar ist.

Bevorzugt ist, daß der Anlenkpunkt an den ersten Schenkel gelegt ist.

Wenn der Betätigungshebel eine Krümmung aufweist, die derjenigen des Stützansatzes entspricht, kann die Verriegelungseinrichtung mit demselben Finger bedient werden, der auch am Stützansatz liegt.

Die Spitze des Instrumentes wird vorteilhaft ebenfalls zum Fassen oder Greifen ausgestaltet, indem die Greifeinrichtungen aus Abschnitten mit stumpfer Zahnung aufweisen, wobei diese Abschnitte im wesentlichen kreisförmig ausgebildet sind.

Im folgenden soll die Erfindung lediglich beispielhaft anhand der beigefügten Zeichnungen näher beschrieben werden. Es zeigt
- Figur 1: eine schematische Darstellung eines Instrumentes gemäß der vorliegenden Erfindung, teilweise aufgerissen;
- Figur 2: eine Detaildarstellung des Schenkels des Instrumentes aus Figur 1, der für den Eingriff des Daumens eines Benutzers gedacht ist ;
- Figur 3: eine Detaildarstellung des zweiten Schenkels des Instrumentes aus Figur 1;
- Figur 4: eine vergrößerte Darstellung einer gezahnten Schneide;
- Figur 5: eine Ansicht der Schneide aus Figur 4 von unten;
- Figur 6: eine vergrößerte Darstellung der zweiten, feststehenden Schneide für ein Instrument gemäß der vorliegenden Erfindung und
- Figur 7: eine vergrößerte Darstellung einer Übertragungsstange.
- Figur 8: eine zweite Ausführungsform des Instrumentes der vorliegenden Erfindung als Faßzange;
- Figur 9: eine Detaildarstellung des Schenkels aus Figur 8, der mit dem Daumen des Benutzers betätigt wird;
- Figur 10: eine Detaildarstellung des zweiten Schenkels aus Figur 8;
- Figur 11: eine Darstellung der Verriegelungseinrichtung;
- Figur 12: eine Darstellung des Endabschnittes des Armes der Verriegelungseinrichtung;
- Figur 13: eine Klinkenvorrichtung für einen Schenkel;
- Figur 14: eine Greifbacke der Greifeinrichtung des Instrumentes nach Figur 8 in Seitenansicht;
- Figur 15: die Greifbacke aus Figur 14 in einer Ansicht von unten;
- Figur 16: die zweite Greifbacke der Greifeinrichtung in einer Ansicht von oben; und
- Figur 17: die Greifbacke aus Figur 16 in Seitenansicht.

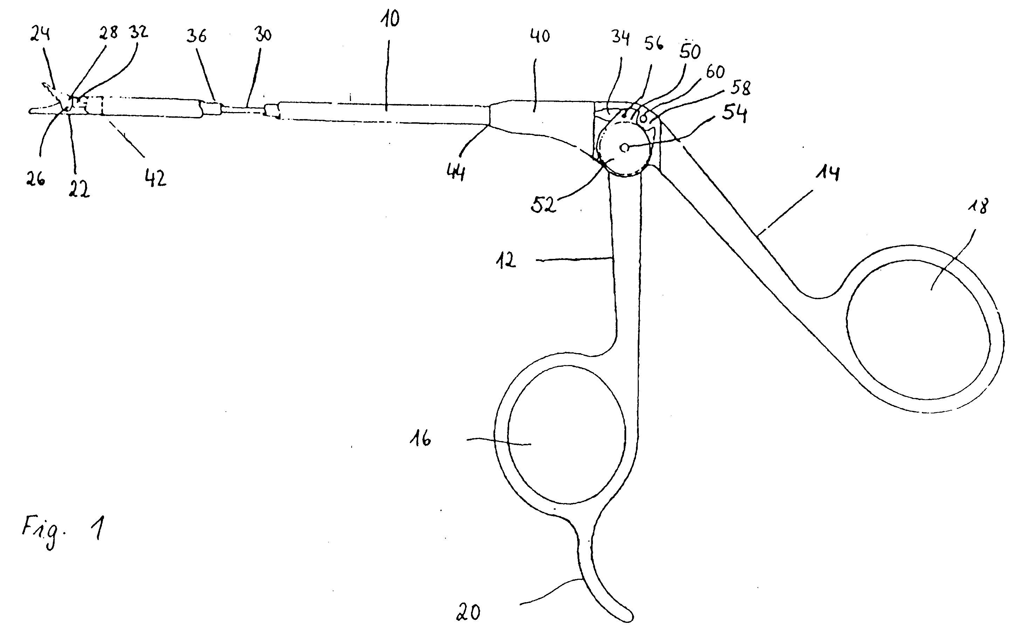

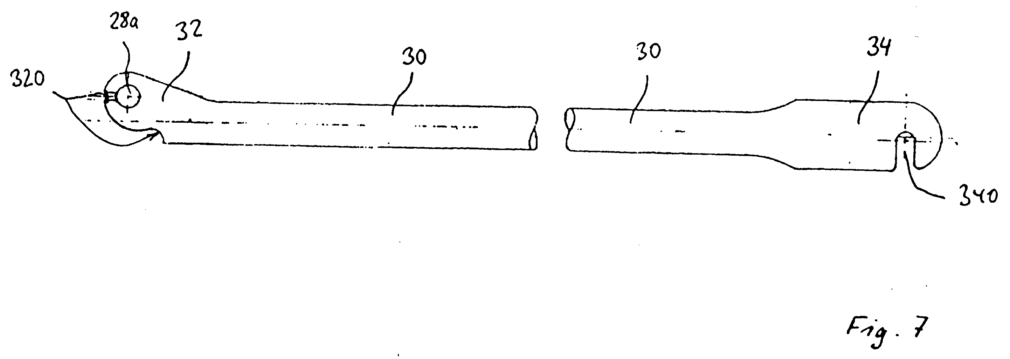

Das in Figur 1 dargestellte Instrument besteht aus einem langgestreckten Rohr 10, an dem die stationären Teile des Instrumentes, nämlich eine feststehende Schneide 22 und ein feststehender Schenkel 14, an einander gegenüberliegenden Enden auf noch näher zu beschreibende Weise befestigt sind. Es ist ein zweiter, bewegbarer Schenkel 12 vorgesehen, der um eine Achse 54 schwenkbar und so gegen den Schenkel 14 bewegbar ist. Beide Schenkel 12, 14 weisen an ihren Enden jeweils ein Auge 16, 18 auf, wobei in das Auge 18 des feststehenden Schenkels 14 üblicherweise der Daumen eines Benutzers eingreift, in das Auge 16 des bewegbaren Schenkels 12 der Zeigefinger, gegebenenfalls auch der Mittelfinger. Am Auge 16 ist ein Stützansatz 20 vorgesehen, der in Richtung auf den Schenkel 14 gekrümmt ist, so daß sich eine Anlagefläche für einen weiteren Finger des Benutzers ergibt. Dieser kann somit die Öffenbewegung der Schenkel 12, 14 gegeneinander unterstützen. An dem dem Auge 16 gegenüberliegenden Ende des Schenkels 12 ist ein Lagerelement 50 vorgesehen, durch das im wesentlichen zentral die Achse 54 geführt ist. Das Lagerelement 50 ist zwischen zwei Scheiben aus reibungsminderndem Material eingebettet, von denen in der Figur lediglich eine Scheibe 52 dargestellt ist. Als Material ist beispielsweise Polytetrafluoräthylen geeignet. Somit sind keine besonderen Reibungskräfte bei der Bewegung der Schenkel 12, 14 gegeneinander zu überwinden und ein Benutzer wird ein besseres Gefühl für die Führung der Schneidbewegung haben. Ferner ist im Kantenbereich des Lagerelementes 50 eine Ausnehmung oder Aussparung 58 vorgesehen, in die ein am Schenkel 14 vorgesehener Anschlagstift 60 eingreift. Durch geeignet gewählte Abmessungen der Ausnehmung 58 kann der Öffnungswinkel bzw. der Schließwinkel der Schenkel 12, 14 eingestellt werden, als Folge davon auch der jeweils entsprechende Winkel zwischen zwei Schneiden 22, 24. Es wird damit einerseits verhindert, daß sich die Schneiden zu weit öffnen, andererseits wird der übermäßigen Belastung eines eine Anlenkachse 56 bildenden Scherstiftes vorgebeugt. Weiterhin ist am Lagerelement 50 eine Übertragungsstange 30 angelenkt, welche in dem langgestreckten Rohr 10 hin- und herbewegbar ist. Die Anlenkung erfolgt über ein im Zusammenhang mit Figur 7 noch genauer zu beschreibendes Anschlußstück 34. Der gesamte Lagerbereich ist von einem Lagergehäuse 40 eingeschlossen, welches hier allerdings teilweise aufgerissen dargestellt ist, wobei die Verbindung des Lagergehäuses 40 zu dem langgestreckten Rohr 10 über eine Schweiß-, Löt- oder Klebverbindung 44 vorgenommen ist. Diese Verbindung kann auch realisiert werden, indem am Innenrohr 10 ein Außengewinde und am Lagergehäuse 40 ein entsprechendes Innengewinde angebracht werden, so daß die beiden Teile miteinander verschraubt werden können. Eine Sicherung der Verbindung erfolgt durch Zugabe eines Spezialklebstoffes.

Wie in dem aufgerissenen Abschnitt des langgestreckten Rohres 10 erkennbar ist, ist die Übertragungsstange 30 von einer Buchse 36 aus reibungsmindernden Material, wiederum beispielsweise Polytetrafluoräthylen, umgeben, so daß sie ebenfalls bewegt werden kann, ohne daß ein größerer Reibungswiderstand zu überwinden wäre. Die Übertragungsstange 30 selbst besteht aus rostfreiem Stahl.

Am distalen Ende des langgestreckten Rohres 10, d. h. an dem Ende, das dem Lagerelement 50 gegenüberliegt, ist die feststehende Schneide 22 mittels einer Schweiß- oder Lötverbindung 42 festgelegt. Auch hier ist eine Klebeverbindung möglich. Die Schneide 22 hat eine sehr geringe Profilhöhe von etwa 1 mm, so daß ein einfacheres Positionieren auch bei festem Gewebe möglich ist. Die bewegbare Schneide 24 ist gegenüber der Schneide 22 um eine Achse 26 verschwenkbar. Über ein Ansatzstück 32 ist die Übertragungsstange 30 weiterhin an der bewegbaren Schneide 24 angelenkt, wobei die Anlenkung mittels eines Stiftes 28 erfolgt. Das Ansatzstück 32 begrenzt, wegen seiner Formgebung mit der geraden vorderen Abschlußkante, die über eine Krümmung in eine oberhalb der Achse 26 verlaufende untere Abschlußkante übergeht, das Schließen der Schneiden 22, 24 über die Unterseite der Schneide 22 hinaus.

Die Figur 1 stellt die Öffnungsstellung der Schenkel 12, 14 dar. Der Anschlagsstift 60 liegt nahezu an einer Kante der Ausnehmung 58 an. Diese Öffnungsstellung entspricht der Öffnungsstellung der Schneiden 22, 24. Wenn jetzt die Schenkel 12, 14 geschlossen werden, bewegt sich das Lagerelement 50 mit der Ausnehmung 58 gegen den Uhrzeigersinn, bis der Anschlagstift 60 an die der ersten Kante gegenüberliegende zweite Kante der Ausnehmung 58 anschlägt. Dabei wird auf die Übertragungsstange 30 eine Druckkraft ausgeübt, welche die Schließbewegung der Schneiden 22, 24 insbesondere der bewegbaren Schneide 24 bewirkt.

Die Achse 54 liegt unterhalb der Achse 56 für die Übertragungsstange 30, um dieser eine Vorschubbewegung für das Schneiden aufzuzwingen.

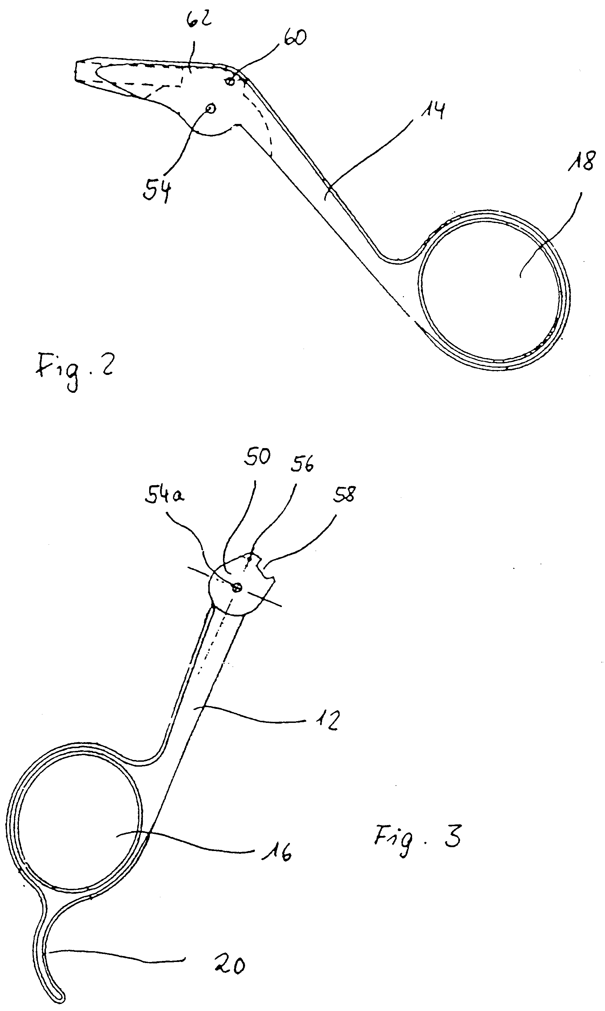

Figur 2 zeigt eine Detaildarstellung des feststehenden Schenkels 14. An einem seiner Enden ist, einstückig mit dem Schenkel 14 ausgebildet, das im wesentlichen elliptische Auge 18 angeformt. Am anderen Ende des Schenkels 14 ist ein Verbindungsteil 62 vorgesehen, auf dem einerseits der Anschlagsstift 60 angeordnet ist, andererseits der Stift 54, der die Achse für die Schwenkbewegung der beiden Schenkel des Instrumentes gegeneinander bildet. Die gegenseitige Lage der Stifte 54, 60 ist so gewählt, daß insbesondere der Anschlagsstift 60 seine Funktion im Wirkzusammenhang mit dem in Figur 2 nicht dargestellten Lagerelement ausüben kann. Das Verbindungsteil 62 ist weiterhin so ausgeformt, daß das langgestreckte Rohr bzw. ein Lagergehäuse daran festgelegt werden kann.

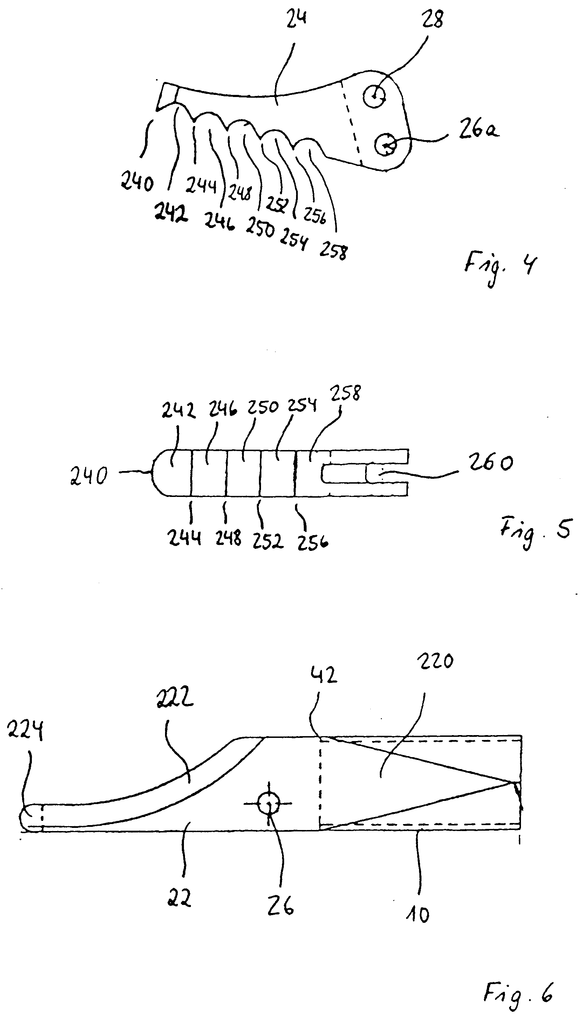

Figur 3 zeigt eine Detaildarstellung des bewegbaren Schenkels 12. Wiederum ist an einem seiner Enden das im wesentlichen elliptische Auge 16 angeformt. Etwa in der Verlängerung des Schenkels 12 ist an dem Auge 16 der Stützansatz 20 ausgebildet, dessen Krümmung so gewählt ist, daß ein Finger des Benutzers in dieser Krümmung an dem Stützanschlag 20 anliegen kann. An dem dem Auge 16 gegenüberliegenden Ende des Schenkels 12 ist das Lagerelement 50 vorgesehen, welches nahezu kreisförmig ausgestaltet ist und zentral eine Öffnung 54a aufweist, welche in bezug auf das Lagerelement 50 die Schwenkachse für die Bewegung der beiden Schenkel gegeneinander definiert. An der von dem Schenkel 12 abgewandten Seite des Lagerelementes 15 ist dieses etwas verlängert ausgebildet und weist eine Ausnehmung 58 mit dem wesentlichen U-förmigen Schnitt auf. Benachbart der Ausnehmung 58 ist der Anlenkpunkt 56 für die Übertragungsstange vorgesehen.

Figur 4 zeigt in Vergrößerung eine Seitenansicht der bewegbaren Schneide 24. Diese ist in ihrem oberen Bereich, also and der Kante, die der feststehenden Schneide abgewandt ist, leicht bogenförmig gekrümmt, der eigentliche Schneidbereich besteht aus fünf Teilbereichen 242, 246, 250, 254, 258, im allgemeinen haben sich vier bis sechs Teilbereiche als günstig erwiesen, die jeweils durch voneinander beabstandete, auch schneidende Zähne 244, 248, 252, 256 voneinander getrennt sind. Auch der Frontbereich der Schneide 24 ist als Zahn 240 ausgebildet. Während sich dieser Zahn 240 allerdings im wesentlichen senkrecht zur Schneidfläche der Schneide 24 erstreckt, sind die Zähne 244, 248, 252, 256 leicht nach hinten, also in Richtung auf die Achse der Schwenkbewegung der Schneiden relativ zueinander, gerichtet. Damit wird erreicht, daß bei der Schneidbewegung die Zähne sukzessiv, beginnend mit dem Zahn 256, das Gewebe in dem zu schneidenden Bereich halten, während der eigentliche Schnitt nacheinander in den Teilbereichen 258, 254, 250, 246, 242 ausgeführt wird. Außerhalb des Bereiches der Einzelschneiden befindet sich eine Öffnung 26a, die für die Schneide 24 die Achse für die Schwenkbewegung definiert, sowie der Anlenkpunkt für die Übertragungsstange, gebildet durch einen durch eine Öffnung geführten Stift 28.

In Figur 5 ist besonders deutlich zu erkennen, welche Maßnahmen für das Festlegen der Übertragungsstange getroffen sind, nämlich eine stufenförmige Ausnehmung 260, auf deren Stufe die Übertragungsstange aufliegen kann. Ebenso ist der gekrümmt verlaufende Frontbereich der Schneide mit dem Zahn 240 deutlich dargestellt. Die Zähne 244, 248, 252, 256 erstrecken sich über die gesamte Breite der Schneide.

Figur 6 zeigt die feststehende Schneide 22 in vergrößerter Darstellung, welche einen Ansatzkeil 220 aufweist, der so ausgelegt ist, daß er in das langgestreckte Rohr 10 eingeführt werden kann, wobei dieses dann mittels einer Schweiß-, Löt- oder Klebverbindung 42 fest mit der Schneide 22 verbunden wird. Vor der Schweiß-, Löt- oder Klebverbindung 42 befindet sich die Achse 26 für die Schwenkbewegung der Schneiden relativ zueinander. Bei der feststehenden Schneide 22 ist eine einheitliche Schneidfläche 222 vorgesehen, welche einen gekrümmten Verlauf hat und sich über die gesamte Höhe der Schneide 22 erstreckt und in einer abgerundeten Front 224 mündet.

Im Querschnitt ist die Schneide 22 quadratisch oder rechteckförmig, was die Stabilität begünstigt und auch einen einfacheren Zusammenbau erlaubt.

Figur 7 zeigt eine Seitenansicht der Übertragungsstange 30 in teilweise gebrochener Darstellung. Ein im wesentlicher langgestreckter Abschnitt weist an seinen Enden 32, 34 Anschlußstücke auf, die jeweils das distale bzw. proximale Ende der Übertragungsstange 30 bewegen. Am proximalen Ende, also am Anschlußstück 34, ist ein vertikaler Schlitz 340 vorgesehen, der sich vom Kantenbereich des Anschlußstückes 34 her in dessen vorderen Bereich bis etwa zum Zentrum hin erstreckt. Diese Ausnehmung wird nach Art eines Hakens auf einen Stift gehängt, der am Anlenkpunkt der Übertragungsstange 30 am Lagerelement vorgesehen ist, was einen einfachen und schnellen Zusammenbau ermöglicht. Am distalen Ende der Übertragungsstange 30, also am Anschlußstück 32, welches leicht abgewinkelt vom langgestreckten Abschnitt der Übertragungsstange 30 verläuft, ist eine Öffnung 28a ausgebildet, durch die ein Stift für die Anlenkung der Übertragungsstange 30 an der bewegbaren Schneide geführt werden kann. Am unteren Teil befindet sich eine Ausnehmung 320, welche auf die im Zusammenhang mit Figur 5 beschriebenen Stufe der dort dargestellten Ausnehmung gesetzt wird.

Der schwächste Teil des Instrumentes ist zweifelsohne der Stift, der die Achse 56 bildet. Dieser sollte so groß wie möglich dimensioniert werden, um Bruch weitgehendst auszuschalten. Insgesamt ist aber zu erwarten, daß beispielsweise wegen der quadratischen bzw. rechteckförmigen Ausgestaltung der Schneide 22, der Übertragungsstange 30 und den entsprechend ausgelegten Verbindungen 42, 44 ein stabiles, unanfälliges Instrument vorliegt.

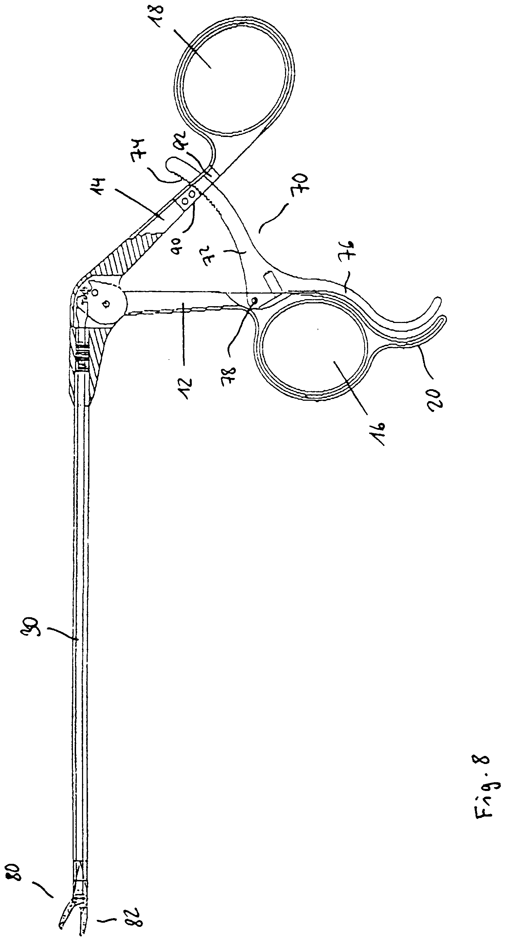

Das in der Figur 8 dargestellte Instrument entspricht in seiner Schließ- und Öffenmechanik dem der Figur 1, jedoch ist, da es als Faß- oder Greifzange verwendet werden soll, am vorderen Ende der Übertragungsstange 30 eine noch genauer zu beschreibende Greifeinrichtung 80, 82 vorgesehen, ferner weist es eine Verriegelungseinrichtung 70 auf, mittels derer die Schenkel 12, 14 in einer bestimmten Position relativ zueinander gehalten werden können.

Die Verriegelungseinrichtung 70 besteht aus einem Arm 72, der kreisbogenförmig gekrümmt und so bemessen ist, daß sein Bogen mindestens den maximalen Öffnungswinkel der Schenkel 12, 14 überstreicht. Der Arm 72 ist an einem seiner Enden an einem Anlenkpunkt 78 am Schenkel 12 schwenkbar angebracht, wobei die Schwenkbewegung mit einem Betätigungshebel 76 durchgeführt wird, der entsprechend der Krümmung des Auges 16 an der an den Schenkel 12 anschließenden Seite und des Stützansatzes 20 geformt ist. Das zweite Ende des Armes 72 ist durch eine Durchgangsöffnung 92 im Schenkel 14 geführt, in der auch eine Klinkenvorrichtung 90 untergebracht ist. Diese weist, dem Arm 72 zugewandt, eine Zahnung auf, die über die Betätigung des Betätigungshebels 76 mit einer Zahnung 74 auf dem Arm 72 in Eingriff bringbar ist. Im Zahnungseingriff ist die Position der Schenkel 12, 14 zueinander fixiert.

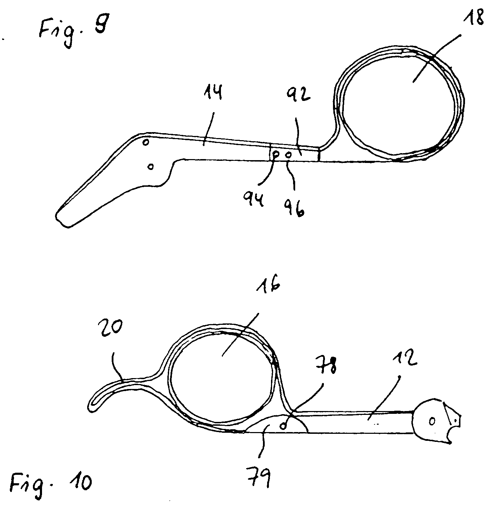

Figur 9 zeigt den Schenkel 14 im Detail. Die Durchgangsöffnung 92 ist in der Nähe des Auges 18 vorgesehen. Die Klinkenvorrichtung wird darin durch Stifte oder dergleichen fixiert, die durch Bohrungen 94, 96 in den die Durchgangsöffnung 92 begrenzenden Wänden vorgesehen sind.

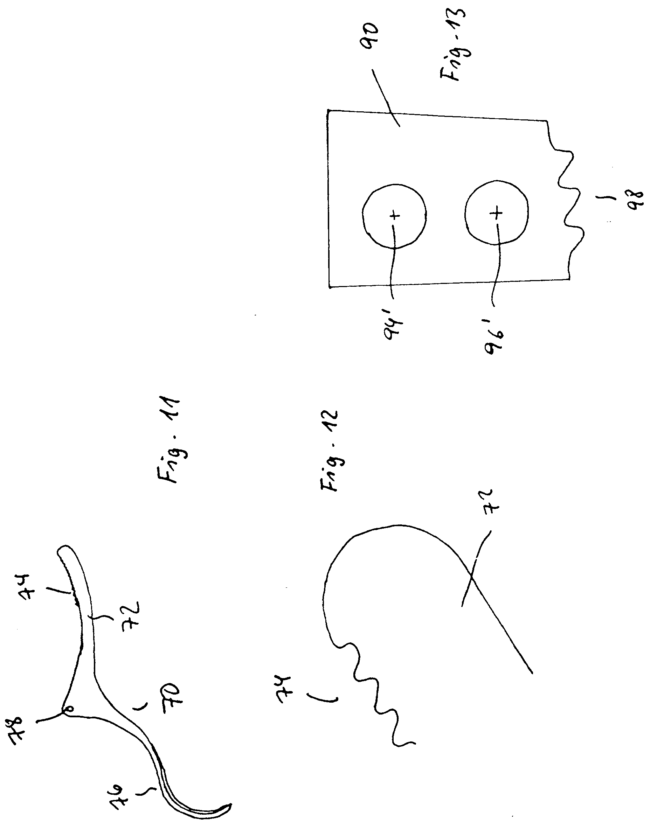

Figur 10 zeigt die Lage des Anlenkpunktes 78 am Schenkel 12. In der Nähe des Auges 16, auf der dem zweiten Schenkel 14 zugewandten Seite, ist eine Ausnehmung 79 mit etwa halbovalem Querschnitt vorgesehen, in die der Anlenkpunkt 76 gelegt ist.

Figur 11 zeigt die Verriegelungseinrichtung 70. Im Übergangsbereich zwischen dem Arm 72 und dem Betätigungshebel 76 ist sie etwa dreieckförmig verbreitert, wobei der Anlenkpunkt 78 an der freien Spitze dieses Dreieckes liegt. Der Arm 72 trägt die Zahnung 74 an einem Endabschnitt, der etwa die Hälfte bis ein Drittel der Länge des Armes 72 beträgt.

Figur 12 zeigt das Ende des Armes 72 mit einem Teil der Zahnung 74. Die Zähne der Zahnung haben abgerundete Spitzen und steil abfallende Flanken auf der dem abgerundeten Ende des Armes 72 abgewandten Seite, flacher abfallende Flanken auf der zugewandten Seite.

In der Figur 13 ist die Klinkenvorrichtung 90 im wesentlichen quaderförmig ausgebildet und trägt an einer ihrer kürzeren Seiten eine Zahnung 98, die in ihrer Formgebung an die zahnung des Armes angepaßt ist, so daß sich zwischen den beiden Zahnungen ein formschlüssiger Eingriff ergibt. Bohrungen 94', 96' sind an Stellen vorgesehen, die denen der Bohrungen 94, 96 der Figur 8 entsprechen.

Figur 14 zeigt eine Greifbacke 80 der Greifeinrichtung, die am Instrument ebenso befestigt wird wie die obere Schneide der Figur 4. Zum Greifen ist eine "stumpfe" Zahnung 84 an der Unterseite der Greifbacke 80 angebracht, wobei die Zahnung 84, wie Figur 15 zeigt, in hintereinanderliegenden kreisförmigen Abschnitten 85, 85', 85'' mit von der Spitze her zunehmendem Durchmesser ausgebildet ist.

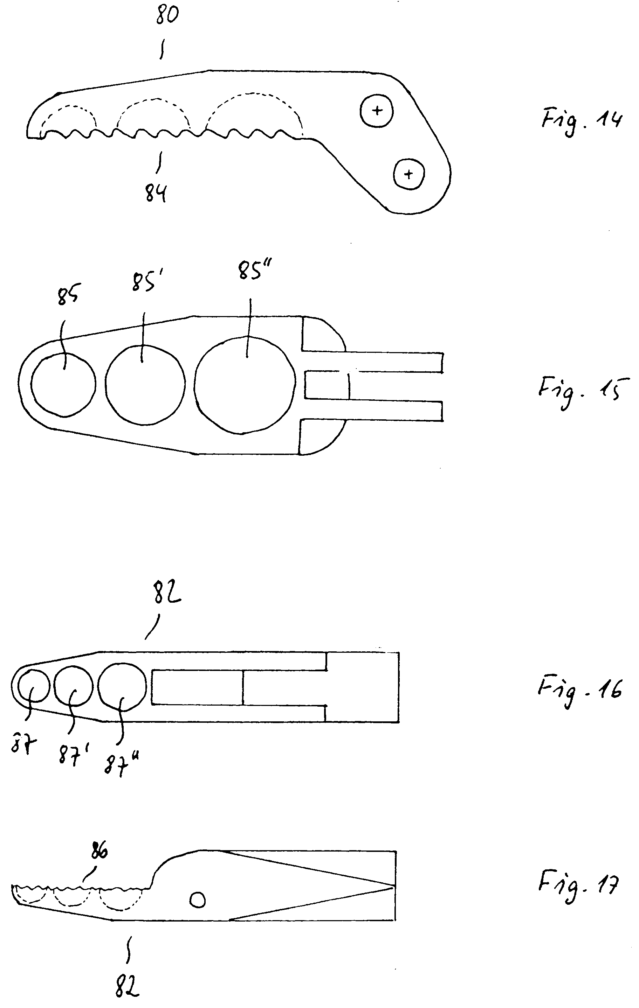

Entsprechend ausgestaltet ist das Gegenstück 82 der Figuren 16 und 17, das die Zahnung 86 in kreisförmigen Abschnitten 87, 87', 87'' ausgebildet hat, wieder hintereinanderliegend und mit von der Spitze her zunehmenden Durchmessern.

### Bezugszeichenliste

- 10: langgestrecktes Rohr
- 12: bewegbarer Schenkel
- 14: feststehender Schenkel
- 16: Auge
- 18: Auge
- 20: Stützansatz
- 22: feststehende Schneide
- 24: bewegbare Schneide
- 26: Achse für die Schwenkbewegung der Schneiden relativ zueinander
- 26a: Öffnung
- 28: Achse für die Anlenkung der Übertragungsstange an der bewegbaren Schneide
- 28a: Öffnung
- 30: Übertragungsstange
- 32: Anschlußstück (distales Ende)
- 34: Anschlußstück (proximales Ende)
- 36: Buchse
- 40: Lagergehäuse
- 42: Schweiß-, Löt- oder Klebeverbindung
- 44: Schweiß-, Löt- oder Klebeverbindung
- 50: Lagerelement
- 52: Scheibe
- 54: Achse für die Schwenkbewegung der Schenkel gegeneinander
- 54a: Öffnung
- 56: Achse für die Anlenkung der Übertragungsstange am Lagerelement
- 58: Ausnehmung am Lagerelement
- 60: Begrenzungsstift
- 62: Verbindungsteil
- 220: Ansatzkeil
- 222: Schneidfläche
- 224: abgerundete Front
- 240: Zahn
- 242: Teilbereich eines Schneidbereiches
- 244: Zahn
- 246: Teilbereich eines Schneidbereiches
- 248: Zahn
- 250: Teilbereich eines Schneidbereiches
- 252: Zahn
- 254: Teilbereich eines Schneidbereiches
- 256: Zahn
- 258: Teilbereich eines Schneidbereiches
- 260: gestufte Ausnehmung
- 320: Aufsatzausnehmung
- 340: vertikaler Schlitz
- 70: Verriegelungseinrichtung
- 72: Arm
- 74: Zahnung
- 76: Betätigungshebel
- 78: Anlenkpunkt
- 80: Greifbacke
- 82: Gegenstück Greifbacke
- 84: Zahnung
- 85, 85', 85'': kreisförmige Abschnitte
- 86: Zahnung
- 87, 87', 87'': kreisförmige Abschnitte
- 90: Klinkenvorrichtung
- 92: Durchgangsöffnung
- 94, 94': Bohrung
- 96, 96': Bohrung
- 98: Zahnung

## Patentansprüche

1. Greif- und/oder Schneidinstrument für endoskopische Zwecke, mit zwei Schenkeln (12, 14), die um eine Achse (54) gegeneinander verschwenkbar sind und die jeweils ein Auge (16, 18) für Finger eines Benutzers aufweisen, wobei einer der Schenkel (12) an einem proximalen Ende einer Übertragungsstange (30) angelenkt ist, wobei zwischen einer Öffnungsstellung und einer Schließstellung gegeneinander bewegbare Greif- und/oder Schneideinrichtungen (22, 24) vorgesehen sind, von denen eine (24) an einem distalen Ende der Übertragungsstange (30) so angelenkt ist, daß die Öffnungsstellung bei Aufbringen einer Zugkraft auf die Übertragungsstange (30) erreicht wird, während die Schließstellung bei Aufbringen einer Druckkraft auf die Übertragungsstange (30) erreicht wird, dadurch gekennzeichnet, daß der an der Übertragungsstange angelenkte Schenkel einen Stützansatz (20) für einen Finger des Benutzers am Auge (16) diese Schenkels aufweist, und daß der Stützansatz (20) in Richtung auf den zweiten Schenkel (14) gekrümmt ist.

2. Greif- und/oder Schneidinstrument nach Anspruch 1, dadurch gekennzeichnet, daß die Übertragungsstange (30) an einem fest mit dem Schenkel (12) verbundenen, um die Achse (54) schwenkbaren Lagerelement (50) angelenkt ist, dessen Schwenkwinkel begrenzt ist.

3. Greif- und/oder Schneidinstrument nach Anspruch 2, dadurch gekennzeichnet, daß das Lagerelement (50) eine Ausnehmung (58) aufweist, in die ein Anschlagstift (60) eingreift.

4. Greif- und/oder Schneidinstrument nach Anspruch 2 oder 3, dadurch gekennzeichnet, daß das Lagerelement (50) beidseitig mit jeweils einer Scheibe (52) aus reibungsminderndem Material wie Polytetrafluoräthylen versehen ist.

5. Greif- und/oder Schneidinstrument nach einem der Ansprüche 1 bis 4, dadurch gekennzeichnet, daß wenigstens eine der Greif- und/oder Schneideinrichtungen (22, 24) gezahnt ist.

6. Greif- und/oder Schneidinstrument nach einem der Ansprüche 1 bis 5, dadurch gekennzeichnet, daß wenigstens eine der Greif- und/oder Schneideinrichtungen (22, 24) eine Vielzahl aneinandergereihter, bogenförmiger schneidender Teilbereiche (242, 246, 250, 254, 258) aufweist, die durch ebenfalls schneidende Zähne (244, 248, 252, 256) voneinander abgesetzt sind.

7. Greif- und/oder Schneidinstrument nach einem der Ansprüche 1 bis 6, dadurch gekennzeichnet, daß die Übertragungsstange (30) in einem langgestreckten Rohr (10) untergebracht ist, in welchem eine die Übertragungsstange (30) im wesentlichen umgebende Buchse (36) aus reibungsminderndem Material wie Polytetrafluoräthylen vorgesehen ist.

8. Greifinstrument nach einem der Ansprüche 1 bis 7, dadurch gekennzeichnet, daß eine Verriegelungseinrichtung (70) vorgesehen ist, welche den ersten Schenkel (12) und den zweiten Schenkel (14) in einer einstellbaren relativen Winkelposition hält.

9. Greifinstrument nach Anspruch 8, dadurch gekennzeichnet, daß die Verriegelungseinrichtung (70) einen ersten Arm (72) aufweist, dessen eines Ende an einem der Schenkel (12, 14) angelenkt ist und dessen anderes Ende eine Klinkenvorrichtung (74) trägt, die mit einer zweiten, kongruent ausgebildeten Klinkenvorrichtung (90) an dem anderen der Schenkel (14, 12) in Eingriff bringbar ist.

10. Greifinstrument nach Anspruch 8 oder 9, dadurch gekennzeichnet, daß die Klinkenvorrichtung (74) eine Zahnung ist.

11. Greifinstrument nach einem der Ansprüche 8 bis 10, dadurch gekennzeichnet, daß in dem die zweite Klinkenvorrichtung (90) aufweisenden Schenkel (14) eine Durchgangsöffnung (92) ausgebildet ist, in die die zweite Klinkenvorrichtung (90) eingelassen ist und die so dimensioniert ist, daß zumindest die Klinkenvorrichtung (74) am Arm (72) durch den freibleibenden Teil der Durchgangsöffnung geführt werden kann.

12. Greifinstrument nach einem der Ansprüche 8 bis 11, dadurch gekennzeichnet, daß die zweite Klinkenvorrichtung (90) eine Zahnung aufweist.

13. Greifinstrument nach einem der Ansprüche 8 bis 12, dadurch gekennzeichnet, daß die Verriegelungseinrichtung (70) einen Betätigungshebel (76) aufweist, mittels dessen der Arm (72) um seinen Anlenkpunkt (78) verschwenkbar ist.

14. Greifinstrument nach einem der Ansprüche 8 bis 13, dadurch gekennzeichnet, daß der Anlenkpunkt (78) an den ersten Schenkel (12) gelegt ist.

15. Greifinstrument nach einem der Ansprüche 8 bis 14, dadurch gekennzeichnet, daß der Betätigungshebel (76) eine Krümmung aufweist, die derjenigen des Stützansatzes (20) entspricht.

16. Greifinstrument nach einem der Ansprüche 8 bis 15, dadurch gekennzeichnet, daß die Greifeinrichtungen (80, 82) aus Abschnitten mit stumpfer Zahnung aufweisen, wobei diese Abschnitte im wesentlichen kreisförmig ausgebildet sind.

## Claims

1. Gripping and/or cutting instrument for endoscopic purposes, with two handles (12,14) which can swivel relative to one another about an axis (54) and which have respectively a ring (16,18) for a user's finger, one of the handles (12) being coupled at a proximal end of a transmission rod (30) and gripping and/or cutting devices (22, 24) movable relative to one another between an open position and a closed position being provided, one of the devices (24) being coupled at a distal end of the transmission rod (30) in such a way that the open position is achieved by exerting a tractive force on the transmission rod (30), whereas the closed position is achieved by exerting a compressive force on the transmission rod (30), characterised in that the handle coupled to the transmission rod has a finger rest (20) for a user's finger on the ring (16) of this handle, and that the said finger rest (20) is curved in the direction of the second handle (14).

2. Gripping and/or cutting instrument according to claim 1, characterised in that the transmission rod (30) is coupled to a bearing element (50) which can swivel by a limited angle about the axis (54) and which is rigidly connected to the handle (12).

3. Gripping and/or cutting instrument according to claim 2, characterised in that the bearing element (50) has a recess (58) in which a stop pin (60) engages.

4. Gripping and/or cutting instrument according to claim 2 or 3, characterised in that the bearing element (50) is provided on both sides with a disc (52) of friction-reducing material such as polytetrafluoroethylene.

5. Gripping and/or cutting instrument according to one of claims 1 to 4, characterised in that at least one of the gripping and/or cutting devices (22, 24) is toothed.

6. Gripping and/or cutting instrument according to one of claims 1 to 5, characterised in that at least one of the gripping and/or cutting devices (22, 24) has a plurality of successive, arcuate cutting regions (242, 246, 250, 254, 258) spaced apart by cutting teeth (244, 248, 252, 256).

7. Gripping and/or cutting instrument according to one of claims 1 to 6, characterised in that the transmission rod (30) is mounted in an elongated tube (10), in which is provided a sleeve (36) of friction-reducing material such as polytetrafluoroethylene which substantially surrounds the said transmission rod (30).

8. Gripping instrument according to one of claims 1 to 7, characterised in that a locking device (70) is provided which holds the first handle (12) and the second handle (14) in an adjustable relative angular position.

9. Gripping instrument according to claim 8, characterised in that the locking device (70) has a first arm (72), one of whose ends is coupled to one of the handles (12, 14) and whose other end carries a ratchet device (74) that can be brought into engagement with a second, congruently shaped ratchet device (90) on the other of the handles (14, 12).

10. Gripping instrument according to claim 8 or 9, characterised in that the ratchet device (74) is a toothing.

11. Gripping instrument according to one of claims 8 to 10, characterised in that a passage opening (92) is formed in the handle (14) having the second ratchet device (90), into which opening the second ratchet device (90) is inserted and which is so dimensioned that at least the ratchet device (74) on the arm (72) can be passed through the free part of the passage opening.

12. Gripping instrument according to one of claims 8 to 11,characterised in that the second ratchet device (90) has a toothing.

13. Gripping instrument according to one of claims 8 to 12, characterised in that the locking device (70) has an actuating lever (76) by means of which the arm (72) can swivel about its coupling point (78).

14. Gripping instrument according to one of claims 8 to 13, characterised in that the coupling point (78) is located on the first handle (12).

15. Gripping instrument according to one of claims 8 to 14, characterised in that the actuating lever (76) has a curvature corresponding to that of the finger rest (20).

16. Gripping instrument according to one of claims 8 to 15, characterised in that the gripping devices (80, 82) have sections with blunt teeth, these sections being substantially circular in shape.

## Revendications

1. Instrument de préhension et/ou de coupe à des fins endoscopiques, comportant deux branches (12, 14), qui peuvent pivoter l'une par rapport à l'autre autour d'un axe (54) et qui présentent chacune un anneau (16, 18) pour des doigts d'un utilisateur, l'une (12) des branches étant articulée sur une extrémité proximale d'une tige de transmission (30), tandis qu'il est prévu des dispositifs de préhension et/ou de coupe (22, 24) mobiles l'un par rapport à l'autre entre une position ouverte et une position fermée, l'un (24) d'eux étant articulé sur une extrémité distale de la tige de transmission (30) de telle manière que la position ouverte soit obtenue lors de l'application d'une force de traction sur la tige de transmission (30) et que la position fermée soit obtenue lors de l'application d'une pression sur la tige de transmission (30), caractérisé en ce que la branche articulée sur la tige de transmission présente un appendice d'appui (20) pour un doigt de l'utilisateur, contre l'anneau (16) de cette branche et en ce que l'appendice d'appui (20) est recourbé en direction de la seconde branche (14).

2. Instrument de préhension et/ou de coupe selon la revendication 1, caractérisé en ce que la tige de transmission (30) est articulée sur un élément de support (50), pouvant pivoter autour de l'axe (54) et fermement relié à la branche (12), l'angle de pivotement de cet élément étant limité.

3. Instrument de préhension et/ou de coupe selon la revendication 2, caractérisé en ce que l'élément de support (50) présente un évidement (58) dans lequel pénètre un ergot d'arrêt (60).

4. Instrument de préhension et/ou de coupe selon la revendication 2 ou 3, caractérisé en ce que l'élément de support (50) est pourvu des deux côtés de chaque fois un disque (52) en un matériau diminuant le frottement, tel que du polytétrafluoroéthylène.

5. Instrument de préhension et/ou de coupe selon l'une des revendications 1 à 4, caractérisé en ce qu'au moins l'un des dispositifs de préhension et/ou de coupe (22, 24) est denté.

6. Instrument de préhension et/ou de coupe selon l'une des revendications 1 à 5, caractérisé en ce qu'au moins l'un des dispositifs de préhension et/ou de coupe (22, 24) présente une pluralité de zones partielles (242, 246, 250, 254, 258) coupantes, en forme d'arc et situées les unes à côté des autres, et séparées les unes des autres par des dents (244, 248, 252, 256) également coupantes.

7. Instrument de préhension et/ou de coupe selon l'une des revendications 1 à 6, caractérisé en ce que la tige de transmission (30) est logée dans un tube très allongé (10), dans lequel il est prévu, pour entourer sensiblement la tige de transmission (30), un fourreau (36) en un matériau diminuant le frottement, tel que du polytétrafluoroéthylène.

8. Instrument de préhension selon l'une des revendications 1 à 7, caractérisé en ce qu'il est prévu un dispositif de verrouillage (70), qui maintient la première branche (12) et la seconde branche (14) dans une position angulaire relative réglable.

9. Instrument de préhension selon la revendication 8, caractérise en ce que le dispositif de verrouillage (70) présente un premier bras (72), dont une extrémité est articulée sur l'une des branches (12, 14) et dont l'autre extrémité porte un mécanisme d'encliquetage (74), qui peut être amené en prise avec un second mécanisme d'encliquetage (90) réalisé de façon correspondante et situé sur l'autre des branches (14, 12).

10. Instrument de préhension selon la revendication 8 ou 9, caractérisé en ce que le mécanisme d'encliquetage (74) est une denture.

11. Instrument de préhension selon l'une des revendications 8 à 10, caractérisé en ce que, dans la seconde branche (14) présentant le second mécanisme d'encliquetage (90), il est prévu une ouverture de passage (92) dans laquelle est logé le second mécanisme d'encliquetage (90) et qui est dimensionnée de telle manière qu'au moins le mécanisme d'encliquetage (74), présent sur le bras (72) puisse être guidé dans la partie, qui reste libre de l'ouverture de passage.

12. Instrument de préhension selon l'une des revendications 8 à 11, caractérisé en ce que le second mécanisme d'encliquetage (90) présente une denture.

13. Instrument de préhension selon l'une des revendications 8 à 12, caractérisé en ce que le dispositif de verrouillage (70) présente un levier d'actionnement (76), au moyen duquel le bras (72) peut être amené à pivoter autour de son point d'articulation (78).

14. Instrument de préhension selon l'une des revendications 8 à 13, caractérisé en ce que le point d'articulation (78) est situé sur la première branche (12).

15. Instrument de préhension selon l'une des revendications 8 à 14, caractérisé en ce que le levier d'actionnement (76) présente une courbure, qui correspond à celle de l'appendice d'appui (20).

16. Instrument de préhension selon l'une des revendications 8 à 15, caractérisé en ce que les dispositifs de préhension (80, 82) présentent des parties à denture non coupante, ces parties étant sensiblement de forme circulaire.
